# EUROPEAN PATENT APPLICATION

(11) **EP 3 358 579 A1**
(43) Date of publication of application: **08.08.2018**
(21) Application number: 17193883.0
(22) Date of filing: 28.09.2017
(51) Int. Cl.: H01F 1/153, G01N 33/00, H01J 37/285

(54) **ANALYSIS METHOD OF COMPOSITION NETWORK TOPOLOGY STRUCTURE AND ANALYSIS PROGRAM THEREOF**

(30) Priority: 28.12.2016 JP 2016255524
(71) Applicant: TDK Corporation, Tokyo 108-0023 (JP)
(72) Inventor: YONEZAWA, Yu, Tokyo, 108-0023 (JP); YOSHIDOME, Kazuhiro, Tokyo, 108-0023 (JP); YOKOTA, Hideaki, Tokyo, 108-0023 (JP); MATSUMOTO, Hiroyuki, Tokyo, 108-0023 (JP); GOTO, Syota, Tokyo, 108-0023 (JP); GOHARA, Takehiro, Tokyo, 108-0023 (JP)
(74) Representative: Epping - Hermann - Fischer

(57) **Abstract**

An analysis method of a composition network topology structure includes obtaining a three-dimensional body for analysis capable of being used for three-dimensional measurement of a concentration distribution of a specific element contained in a sample within a predetermined measurement range. The three-dimensional body for analysis is divided into unit grids composed of a plurality of finer three-dimensional bodies. An amount of the specific element contained in each of the unit grids is obtained. Maximum-point grids respectively having a largest amount of the specific element among adjacent unit grids are obtained. The composition network topology structure of the specific element owned by the sample is quantified in relation to the maximum-point grids contained in the three-dimensional body for analysis.

## Description

### BACKGROUND OF THE INVENTION

### 1. Field of the Invention

The present invention relates to an analysis method of a composition network topology structure and an analysis program thereof.

### 2. Description of the Related Art

Low power consumption and high efficiency have been demanded in electronic, information, communication equipment, and the like. Moreover, the above demands are becoming stronger for a low carbon society. It is thus required that characteristics of various kinds of functional materials used for electronic, information, communication equipment, and the like be improved.

For example, magnetic cores used for power supply circuits are required to improve their permeability and reduce their core loss (magnetic core loss). If core loss is reduced, the loss of electric power energy is reduced, and high efficiency and energy saving are achieved.

It is conceived that reducing coercivity of a magnetic material constituting a magnetic core is a method of reducing core loss of the magnetic core. Magnetic materials having a new composition is under development for reduction in coercivity of magnetic materials. Patent Document 1 discloses that a soft magnetic alloy powder having a large permeability and a small core loss and being suitable for magnetic cores is obtained by changing particle shape of a powder.

In functional materials constituting various kinds of electronic devices, however, a method for characteristic improvement in various kinds of electronic devices by analyzing network structures of specific elements is not conventionally under development.

Patent Document 1: JP 2000-30924 A

### SUMMARY OF THE INVENTION

The present invention has been achieved under such circumstances. It is an object of the invention to provide an analysis method of a composition network topology structure and an analysis program thereof capable of achieving characteristic improvement in various kinds of functional materials by analyzing a network structure of a specific element.

To achieve the above object, the analysis method of the composition network topology structure according to the present invention is an analysis method of a composition network topology structure, including the steps of:
obtaining a three-dimensional body for analysis capable of being used for three-dimensional measurement of a concentration distribution of a specific element (this term also includes specific compounds) contained in a sample within a predetermined measurement range;
dividing the three-dimensional body for analysis into unit grids composed of a plurality of finer three-dimensional bodies;
obtaining an amount of the specific element contained in each of the unit grids;
obtaining maximum-point grids respectively having a largest amount of the specific element among adjacent unit grids; and
quantifying the composition network topology structure of the specific element owned by the sample in relation to the maximum-point grids contained in the three-dimensional body for analysis.

The amount of the specific element existing inside the three-dimensional body for analysis is measured using a three-dimensional atom probe, for example. The method of the present invention can easily obtain the number of maximum-point grids or so (or coordination number of maximum-point grids, or connection length of each maximum-point grid) based on the measurement data. The degree of the composition network topology structure of the specific element in the sample can be digitized based on the maximum-point grids. If the degree of the composition network topology structure can be digitized, the degree and various characteristics such as magnetic properties of the sample can be linked, and the digitalization can be effectively utilized as an assistance of material development.

That is, the analysis can be carried out by relating the degree of the composition network topology structure of the specific element and various characteristics such as magnetic properties owned by the sample. Instead, it is also possible to achieve optimization between the degree of the composition network topology structure of the specific element and a manufacturing method for preparation of the sample.

The analysis method of the composition network topology structure may further include the steps of:
forming virtual connection lines by linking a plurality of centers of the maximum-point grids existing inside the three-dimensional body for analysis;
forming final virtual connection lines by deleting the virtual connection lines being crossed based on a predetermined rule; and
determining the number of the final virtual connection lines linking each of the maximum-point grids as a coordination number,
wherein the composition network topology structure of the specific element owned by the sample may be quantified based on the coordination number.

The method can easily automatically calculate coordination number using a computer program, for example. The degree of the composition network topology structure of the specific element can be easily quantified (digitized) by calculating the number of maximum-point grids having a predetermined number or more of coordination number.

The analysis method of the composition network topology structure may further include the steps of:
forming virtual connection lines by linking a plurality of centers of the maximum-point grids existing inside the three-dimensional body for analysis;
forming final virtual connection lines by deleting the virtual connection lines being crossed based on a predetermined rule; and
obtaining data of the virtual connection lines including at least one of a total length of the final virtual connection lines linking the maximum-point grids inside the three-dimensional body for analysis, an average distance of the final virtual connection lines, a standard deviation of the final virtual connection lines, and an existence ratio of the final virtual connection lines within a predetermined length,
wherein the composition network topology structure of the specific element owned by the sample may be quantified based on the data of the virtual connection lines.

Even such a method can easily automatically calculate the data of virtual connection lines. The degree of the composition network topology structure of the specific element can be easily quantified (digitized) by calculating the data of virtual connection lines having predetermined conditions.

The analysis method of the composition network topology structure may further include a step of obtaining an average value of the amount of the specific element contained in each of the unit grids with respect to the entire three-dimensional body for analysis,
wherein the entire three-dimensional body for analysis may be divided into a high-concentration region having continuous unit grids whose amount is larger than a threshold value determined as the average value and a low-concentration region having continuous unit grids whose amount is equal to or smaller than the threshold value, and
the step of deleting the virtual connection lines may delete virtual connection lines passing through the low-concentration region.

Such a method can easily prepare final virtual lines related to a network and easily prepare the above-mentioned data of coordination number or virtual connection lines. As a result, the degree of the composition network topology structure of the specific element can be easily quantified.

An analysis program according to the present invention is configured to implement any of the above-mentioned analysis methods of the composition network topology structure at the time of implementation in a programmable computer.

### BRIEF DESCRIPTION OF THE DRAWINGS

FIG. 1 is a photograph of a Fe concentration distribution of a soft magnetic alloy applied with an analysis method according to an embodiment of the present invention using a three-dimensional atom probe.
FIG. 2 is a model diagram of a network structure owned by the soft magnetic alloy shown in FIG. 1.
FIG. 3A is a schematic view of a step of searching maximum-point grids in an analysis method according to an embodiment of the present invention.
FIG. 3B is a schematic view of a step related to FIG. 3A.
FIG. 4 is a schematic view of a state where line segments linking centers of all maximum-point grids are formed.
FIG. 5 is a schematic view of a state where the schematic view shown in FIG. 4 is divided into a region whose Fe content is more than its average content and a region whose Fe content is equal to or less than its average content.
FIG. 6 is a schematic view showing a continuous step from FIG. 5.
FIG. 7 is a schematic view showing a continuous step from FIG. 6.
FIG. 8 is a graph showing a relation between a coordination number and a maximum-point number ratio in Examples of the present invention.
FIG. 9 is a graph showing a relation between a length of a virtual connection line and a ratio of the number of the virtual connection lines in Examples of the present invention.

### DESCRIPTION OF THE PREFERRED EMBODIMENTS

Hereinafter, the present invention will be described based on embodiments shown in the figures.

### First Embodiment

The present embodiment uses a soft magnetic alloy whose main component is Fe as a sample and describes an embodiment of implementation of an analysis method of a Fe composition network topology structure. The analysis method of the present embodiment is workable for programmable computers. The program may be communicable via the internet or so, or may be memorized into storage media, such as hard disc, and implemented by the computers or so.

First, the Fe composition network topology structure (hereinafter may be simply referred to as a network structure) owned by the soft magnetic alloy will be described.

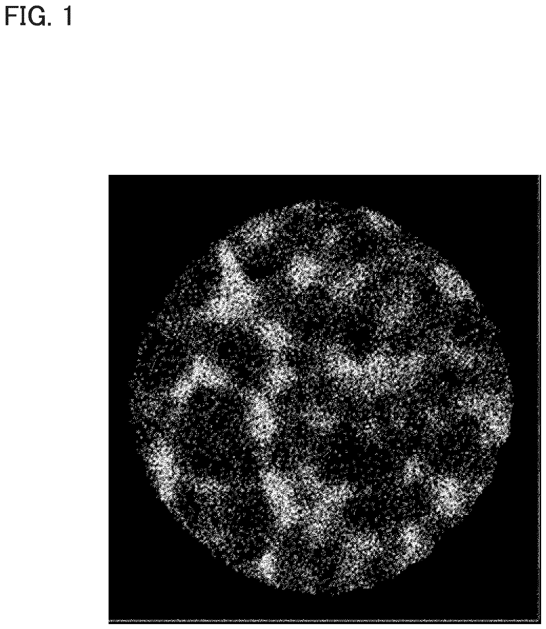

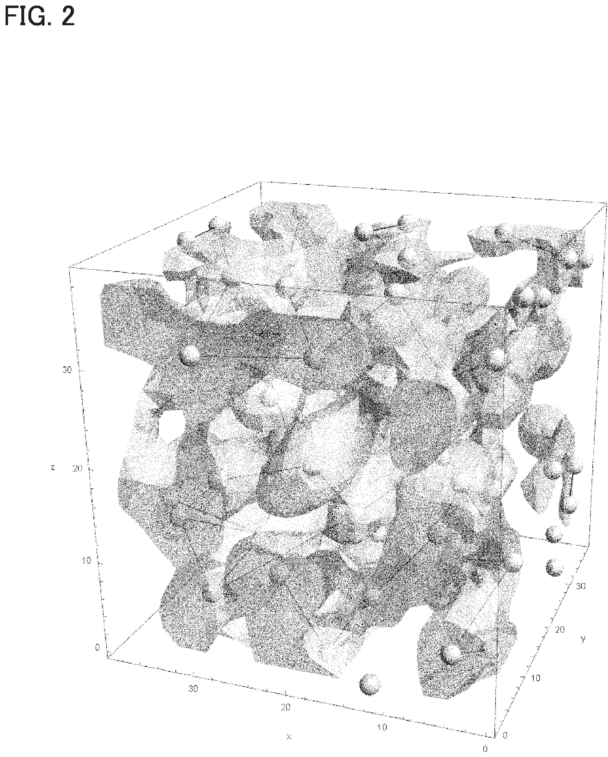

The Fe composition network topology structure is a structure where phases whose Fe content is higher than that of an average composition of the soft magnetic alloy are connected to each other in network. When observing a Fe concentration distribution of the soft magnetic alloy according to the present embodiment using a three-dimensional atom probe (hereinafter, a three-dimensional atom probe may be represented as a 3DAP) with a thickness of 5 nm, it can be observed that portions having a high Fe content are distributed in network as shown in FIG. 1. FIG. 2 is a schematic view obtained by three-dimensionalizing this distribution. Incidentally, FIG. 1 is an observation result of Sample No. 39 in Examples mentioned below using a 3DAP.

In conventional soft magnetic alloys containing Fe, a plurality of portions having a high Fe content respectively has a spherical shape or an approximately spherical shape and exists at random via portions having a low Fe content. In the soft magnetic alloy according to the present embodiment, portions having a high Fe content are linked in network and distributed as shown in FIG. 2.

As described below, an aspect of the Fe composition network topology structure can be quantified by measuring the number of maximum points and/or coordination number of maximum points of the Fe composition network structure.

The maximum point of the Fe composition network topology structure is a point whose Fe content is locally higher than that of its surroundings. The coordination number of the maximum point is the number of the other maximum points linking to a maximum point via the Fe composition network topology structure.

Hereinafter, an analysis procedure of the Fe composition network topology structure according to the present embodiment will be described using the figures, and a maximum point, a coordination number of the maximum point, and a calculation method thereof will be thereby described.

First, a sample to be analyzed is prepared, and a cube (three-dimensional body for analysis) in the sample whose one side preferably has a length of 20 nm or more, for example 40 nm, is determined as a measurement range. Then, this cube is divided into cubic grids (unit grids) whose one side preferably has a length of 0.2 nm or more, more preferably has a length of 0.2 nm or more that is 1/10 or less of a length of the one side of the sample. For example, this cube is divided into cubic grids of 1 nm. When a cube of 40 nm is divided into cubic grids of 1 nm, 64,000 grids (40×40×40 = 64,000) exist in one measurement range.

Next, Fe is selected as a specific element contained in each unit grid, and a Fe content is evaluated. Then, an average value (hereinafter may be represented as a threshold value) of the Fe contents in all unit grids is calculated. An average value of the Fe contents is expected to be a value that is substantially equivalent to a value calculated from an average composition of each soft magnetic alloy.

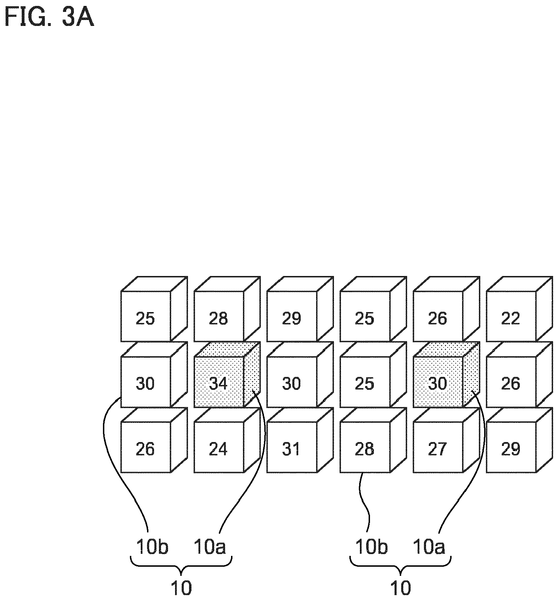

Next, a grid whose Fe content exceeds the threshold value and is higher than that of all adjacent unit grids is determined as a maximum-point grid. FIG. 3A shows a model showing a step of searching the maximum-point grids. Numbers written inside each unit grid 10 represent a Fe content in each grid. Maximum-point grids 10a are determined as a unit grid 10 whose Fe content is equal to or larger than Fe contents of all adjacent grids 10b.

Incidentally, FIG. 3A shows eight adjacent grids 10b with respect to a single maximum-point grid 10a, but in fact nine adjacent grids 10b also exist respectively front and back the maximum points 10a of FIG. 3A. That is, 26 adjacent grids 10b exist with respect to a single maximum-point grid 10a.

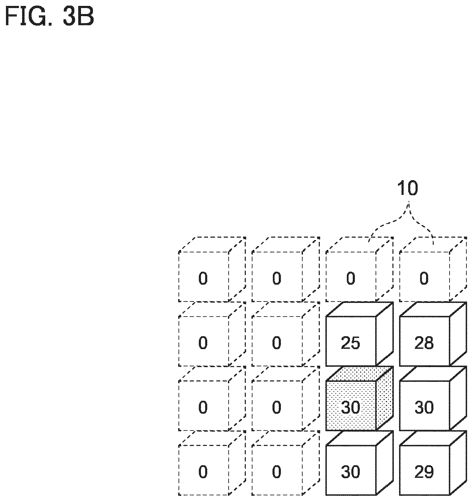

As shown in FIG. 3B, with respect to grids 10 located at the end of the measurement range, grids whose Fe content is zero are considered to exist outside the measurement range.

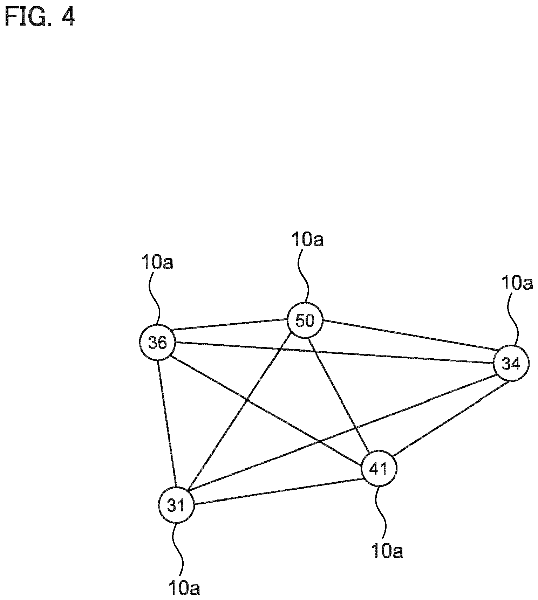

Next, as shown in FIG. 4, line segments (virtual connection lines) linking all of the maximum-point grids 10a contained in the measurement range are drawn. When drawing the line segments, centers of the grids 10a are connected to each other. Incidentally, the maximum-point grids 10a are represented as circles for convenience of description in FIG. 4 to FIG. 7. Numbers written inside the circles represent a Fe content.

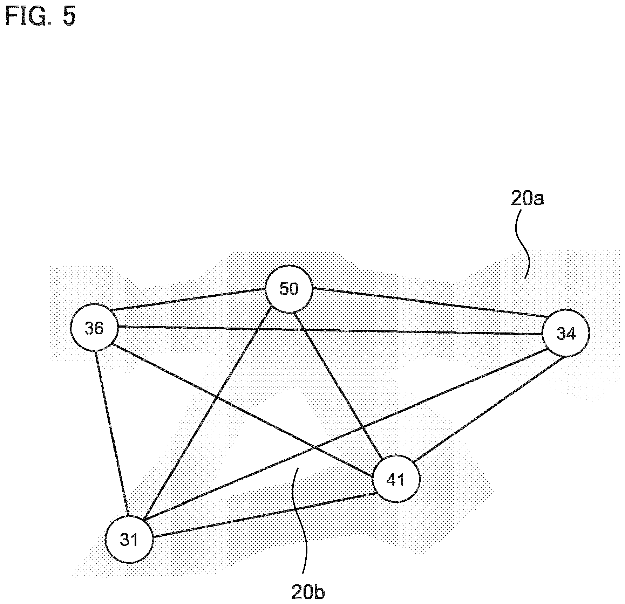

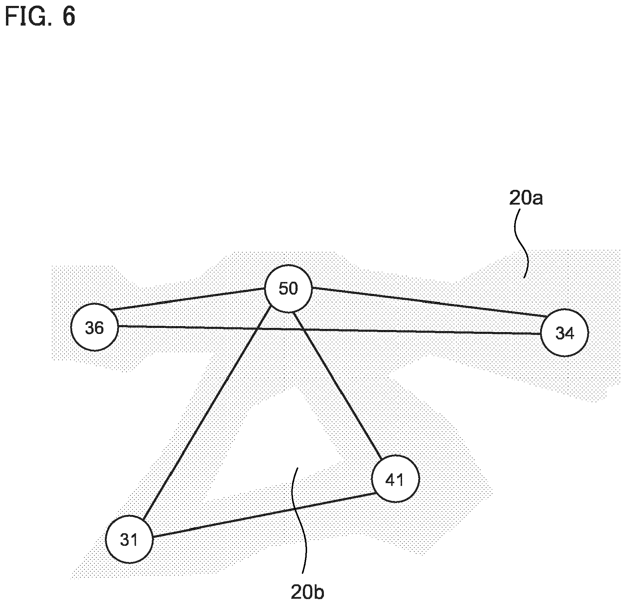

Next, as shown in FIG. 5, the measurement range is divided into a high-concentration region 20a where unit grids whose Fe content is higher than a threshold value are continuous and a low-concentration region 20b where unit grids whose Fe content is equal to or lower than a threshold value are continuous. A network structure of the high-concentration region 20a is the Fe composition network topology structure. Then, as shown in FIG. 6, line segments passing through the low-concentration region 20b are deleted.

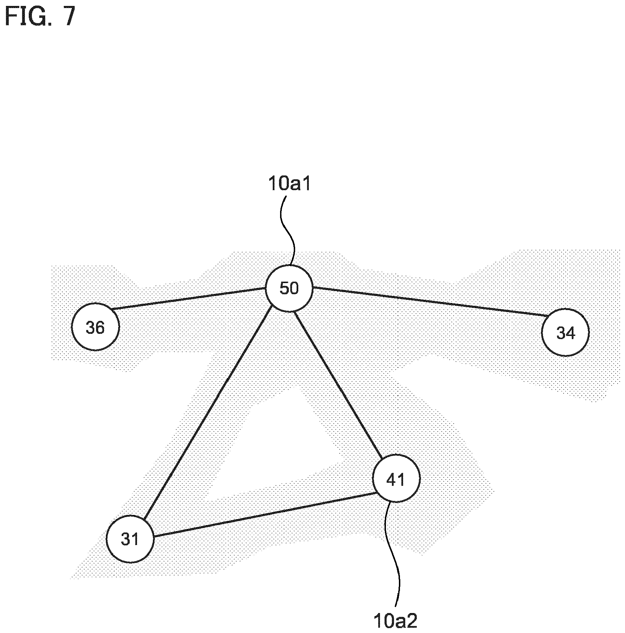

Next, as shown in FIG. 7, when no low-concentration region 20b exists inside a triangle formed by the line segments, the longest line segment of three line segments constituting this triangle is deleted. Incidentally, when the low-concentration region 20b exists inside the triangle, line segments of the triangle are not deleted. As a result, final virtual connection lines as line segments finally obtained do not cross each other and link each of the adjacent maximum-point grids by single line segments, and these line segments do not pass through the low-concentration region 20b.

Then, the number of final virtual connection lines extending from each maximum point 10a is determined as a coordination number of each maximum point 10a. For example, in FIG. 7, a maximum point 10a1 whose Fe content is 50 has a coordination number of 4, and a maximum point 10a2 whose Fe content is 41 has a coordination number of 2.

When a grid existing on an outermost surface within a measurement range of the cube for analysis as the three-dimensional body for analysis is a maximum-point grid, this maximum-point grid is excluded from calculation of a ratio of maximum points whose coordination number is within a predetermined range mentioned below.

Incidentally, the Fe composition network topology structure also includes a maximum point whose coordination number is zero and a region whose Fe content is higher than a threshold value existing in the surroundings of a maximum point whose coordination number is zero.

The analysis method shown above can sufficiently highly improve accuracy of calculation results by conducting the analysis several times in respectively different measurement ranges. Preferably, the analysis is conducted three times or more in respectively different measurement ranges.

In the analysis method of the Fe composition network topology structure according to the present embodiment, for example, the above-mentioned analysis method can confirm that favorable magnetic properties are obtained if there exist 400,000/µm³ or more maximum-point grids whose Fe content is locally higher than that of their surroundings.

In the present embodiment, it can be confirmed that favorable magnetic properties are obtained if a ratio of maximum-point grids whose coordination numbers are 1 or more and 5 or less to all maximum-point grids is 80% or more and 100% or less. Incidentally, a denominator of the ratio of the maximum-point grids is a total number of all maximum-point grids existing in the entire measurement range. When coordination number is evaluated, however, the maximum-point grids of the denominator exclude maximum-point grids existing on the surface of the end of the evaluation region.

The analysis method of the present embodiment has found that a soft magnetic alloy having predetermined magnetic properties is obtained by having a Fe composition network topology structure where the number of maximum points is equal to or more than a predetermined value and a ratio of maximum points whose coordination numbers are 1 or more and 5 or less is within a predetermined range. That is, it has been found that a soft magnetic alloy having a low coercivity and a high permeability and excelling in soft magnetic properties particularly in high frequencies can be obtained.

Moreover, a new knowledge shown below has been obtained using the analysis method of the present embodiment. That is, it has been found that a soft magnetic alloy having a low coercivity and a high permeability and excelling in soft magnetic properties particularly in high frequencies can be obtained when a volume ratio of the Fe composition network topology structure occupied in the entire soft magnetic alloy is 25 vol% or more and 50 vol% or less, particularly 30 vol% or more and 40 vol% or less. Incidentally, the volume ratio of the Fe composition network topology structure is a volume ratio of the region 20a whose Fe content is higher than a threshold value to a total of the region 20a whose Fe content is higher than a threshold value and the region 20b whose Fe content is equal to or lower than a threshold value.

Moreover, a new knowledge shown below has been obtained using the analysis method of the present embodiment. When comparing a Fe-Si-M-B-Cu-C based soft magnetic alloy with a Fe-M-B-C based soft magnetic alloy, the Fe-M-B-C based soft magnetic alloy tends to have a higher number of maximum points and also have a larger coordination number.

As mentioned above, the analysis method of the present embodiment can easily obtain the number of maximum-point grids (or coordination number of maximum-point grids) or so. The degree of the composition network topology structure of specific elements in the sample can be digitized based on the maximum-point grids. If the degree of the composition network topology structure can be digitized, the degree and various characteristics such as magnetic properties of the sample can be linked, and the digitalization can be effectively utilized as an assistance of material development.

That is, the analysis can be carried out by relating the degree of the composition network topology structure of specific elements and various characteristics such as magnetic properties owned by the sample. Instead, it is also possible to achieve optimization between the degree of the composition network topology structure of specific elements and a manufacturing method for preparation of the sample.

The analysis method of the present embodiment can easily automatically calculate coordination number using a computer program, for example. The degree of the composition network topology structure of specific elements can be easily quantified (digitized) by calculating the number of maximum-point grids having a predetermined number or more of coordination number.

It can be expected that the development speed of magnetic materials having required characteristics such as specific magnetic properties is further improved by implementing the analysis method of the present embodiment in a computer program.

### Second Embodiment

Hereinafter, an analysis procedure of a Fe composition network phase according to Second Embodiment of the present invention will be described. This embodiment describes an analysis method of a degree of network formation digitized by calculating a total distance of virtual connection lines (hereinafter simply referred to as "virtual lines") and/or an average distance of the virtual lines. Incidentally, the following description will not partially describe parts common to First Embodiment and describe parts different from First Embodiment in detail.

First, a sample to be analyzed is prepared, and a cube (three-dimensional body for analysis) in the sample is divided into unit grids having a predetermined size. This is the same as First Embodiment.

Next, a Fe content in each grid is evaluated. This is also the same as First Embodiment. Then, an average value (hereinafter may be referred to as a threshold value) of the Fe contents in all of the grids is calculated. This is also the same as First Embodiment.

As shown in FIG. 3A to FIG. 7, maximum points of the grids are obtained, and line segments linking all of maximum points 10a contained in a measurement range are drawn. This is also the same as First Embodiment. In the present embodiment, however, the line segments linking all of the maximum points 10a are referred to as virtual lines. Line segments linking between a maximum point of a unit grid existing on the outermost surface in the measurement range and another maximum point existing on the same outermost surface are deleted. When calculating a virtual-line average distance and a virtual-line standard deviation mentioned below, virtual lines passing through maximum points of grids existing on the outermost surface are excluded from this calculation.

The analysis can be implemented due to calculation of a virtual-line total distance obtained by summing up lengths of final virtual lines remaining in the measurement range. Moreover, the analysis can be also implemented due to calculation of the number of the final virtual lines and a virtual-line average distance. The virtual-line average distance is a distance per one final virtual line. The analysis can be also implemented due to calculation of a standard variation of the virtual-line average distance and an existence ratio of virtual lines having a predetermined length.

Incidentally, the Fe composition network phase also includes a maximum point having no final virtual lines and a region existing in its surroundings and having a Fe content that is higher than a threshold value.

In the analysis method shown above, results to be calculated can be sufficiently precise by carrying out the measurement several times, preferably three or more times, in respectively different measurement ranges.

The above-mentioned analysis method of the Fe composition network topology structure according to the present embodiment can confirm that a soft magnetic alloy having favorable magnetic properties can be achieved when the virtual-line total distance is 10 mm to 25 mm per soft magnetic alloy 1 µm³. The above-mentioned analysis method of the Fe composition network topology structure according to the present embodiment can also confirm that a soft magnetic alloy having favorable magnetic properties can be achieved when the virtual-line average distance, that is, the average of the distances of the virtual lines is 6 nm or more and 12 nm or less.

The above-mentioned analysis method according to the present embodiment can confirm that a soft magnetic alloy having a low coercivity and a high permeability and excelling in soft magnetic properties particularly in high frequencies can be obtained by having a Fe composition network phase whose virtual-line total distance and/or virtual-line average distance is/are within the above range(s).

As mentioned above, the analysis method of the present embodiment can easily obtain virtual line data such as virtual-line total distance and/or virtual-line average distance, which show(s) a connection length of each maximum-point grid. The degree of the composition network topology structure of specific elements in the sample can be digitized based on the virtual line data. If the degree of the composition network topology structure can be digitized, the degree and various characteristics such as magnetic properties of the sample can be linked, and the digitalization can be effectively utilized as an assistance of material development.

That is, the analysis can be carried out by relating the degree of the composition network topology structure of specific elements and various characteristics such as magnetic properties owned by the sample. Instead, it is also possible to achieve optimization between the degree of the composition network topology structure of specific elements and a manufacturing method for preparation of the sample.

The analysis method of the present embodiment can easily automatically calculate the virtual line data using a computer program, for example. The degree of the composition network topology structure of specific elements can be easily quantified (digitized) by calculating the virtual line data having predetermined conditions.

Moreover, the analysis method of the present embodiment can easily prepare final virtual lines related to a network and easily prepare the above-mentioned virtual line data. As a result, the degree of the composition network topology structure of specific elements can be easily quantified.

It can be expected that the development speed of magnetic materials having required characteristics such as specific magnetic properties is further improved by implementing the analysis method of the present embodiment in a computer program.

Incidentally, the present invention is not limited to the above-mentioned embodiments, but may be variously changed within the scope of the present invention.

For example, the above-mentioned embodiments employ a cube whose longitudinal length and lateral length are the same as a three-dimensional body for analysis, but may use a cube whose longitudinal length and lateral length are different from each other. The above-mentioned embodiments also employ a cube whose longitudinal length and lateral length are the same with respect to a unit grid, but may employ a cube whose longitudinal length and lateral length are different from each other. The three-dimensional body for analysis is not limited to a cube, and may be another shape such as a sphere. This is the case with the unit grid.

The above-mentioned embodiments employ a three-dimensional atom probe as a means of three-dimensional measurement of the amount of specific elements existing inside the three-dimensional body for analysis, but the present invention is not limited thereto.

Materials applied with the analysis method of the present invention are not limited to magnetic materials such as the above-mentioned soft magnetic body alloy, and may be applied to any material.

### Examples

Hereinafter, the present invention is described based on further detailed examples, but is not limited to the examples.

### Example 1

Hereinafter, the present invention will be specifically described based on examples.

### (Experiment 1: Sample No. 1 to Sample No. 26)

Each of pure metal materials was weighed so that a base alloy having a composition of Fe: 73.5 atom%, Si: 13.5 atom%, B: 9.0 atom%, Nb: 3.0 atom%, and Cu: 1.0 atom% was obtained. Then, the base alloy was manufactured by evacuating a chamber and thereafter melting the pure metal materials at high frequencies.

Then, the manufactured base alloy was heated and molten to be turned into a metal in a molten state at 1300°C. This metal was thereafter sprayed against a roll by a single roll method at a predetermined temperature and a predetermined vapor pressure, and ribbons were prepared. These ribbons were configured to have a thickness of 20 µm by appropriately adjusting a rotation speed of the roll. Next, each of the prepared ribbons was subjected to a heat treatment, and single-plate samples were obtained.

In Experiment 1, each sample shown in Table 1 was manufactured by changing a temperature of the roll, a vapor pressure, and heat treatment conditions. The vapor pressure was adjusted using an Ar gas whose dew point had been adjusted.

Each of the ribbons before the heat treatment was subjected to an X-ray diffraction measurement for confirmation of existence of crystals. In addition, existence of microcrystals was confirmed by observing a restricted visual field diffraction image and a bright field image at 300,000 magnifications using a transmission electron microscope. As a result, it was confirmed that the ribbons of each example had no crystals or microcrystals and were amorphous.

Then, each sample after each ribbon was subjected to a heat treatment was measured with respect to coercivity, permeability at 1 kHz frequency, permeability at 1 MHz frequency. Table 1 shows the results.

Moreover, each sample was measured with respect to Fe content using a three-dimensional atom probe (3DAP), and the number of maximum points of Fe, a ratio of maximum points whose coordination number was 1 or more and 5 or less, a ratio of maximum points whose coordination number was 2 or more and 4 or less, and a content ratio of the Fe network topology structure to the entire sample were analyzed based on the results of Fe content using a program of the analysis method shown in First Embodiment of the present invention. Table 1 shows the results.

**Table 1**

| Sample No. | Roll temperature (°C) | Vapor pressure in chamber (hPa) | Existence of crystals before heat treatment | Heat treatment conditions | | Network structures | | | | Coercivity (A/m) | *µ*r (1kHz) | *µ*r (1MHz) |
|---|---|---|---|---|---|---|---|---|---|---|---|---|
| | | | | Heat treatment temperature (°C) | Heat treatment time (h) | Number of maximum points (ten thousand / *µ*m³⁾ | Coordination number is 1 or more and 5 or less (%) | Coordination number is 2 or more and 4 or less (%) | Fe network composition phase (vol%) | | | |
| 1 | 70 | 25 | microscrystals | 550 | 1 | **13** | - | - | - | **7.03** | **6200** | **730** |
| 2 | 70 | 18 | amorphous | 550 | 1 | **14** | - | - | - | **1.86** | **63000** | **1900** |
| 3 | 70 | 11 | amorphous | 550 | 1 | 54 | 95 | 76 | 35 | 0.96 | 103000 | 2700 |
| 4 | 70 | 4 | amorphous | 550 | 1 | 67 | 95 | 84 | 36 | 0.85 | 118000 | 2800 |
| 5 | 70 | Ar filling | amorphous | 550 | 1 | 67 | 95 | 84 | 36 | 0.79 | 110000 | 2670 |
| 6 | 70 | vacuum | amorphous | 550 | 1 | 67 | 96 | 82 | 35 | 0.73 | 108000 | 2560 |
| 7 | 70 | 4 | amorphous | 550 | 0.1 | 67 | 66 | 54 | 18 | **1.23** | **52000** | **1800** |
| 8 | 70 | 4 | amorphous | 550 | 0.5 | 72 | 84 | 69 | 31 | 0.82 | 108000 | 2730 |
| 9 | 70 | 4 | amorphous | 550 | 10 | 58 | 96 | 83 | 41 | 0.92 | 103000 | 2570 |
| 10 | 70 | 4 | amorphous | 550 | 100 | **32** | **73** | 48 | 54 | **1.25** | **68000** | 1800 |
| 11 | 70 | 4 | amorphous | 450 | 1 | 5 | - | - | - | **1.40** | **40000** | 1500 |
| 12 | 70 | 4 | amorphous | 500 | 1 | 72 | 84 | 69 | 31 | 0.82 | 108000 | 2730 |
| 13 | 70 | 4 | amorphous | 550 | 1 | 66 | 96 | 83 | 37 | 0.86 | 107000 | 2580 |
| 14 | 70 | 4 | amorphous | 600 | 1 | 58 | 96 | 83 | 41 | 0.94 | 101000 | 2570 |
| 15 | 70 | 4 | amorphous | 650 | 1 | 54 | **70** | 43 | 52 | **48** | **2000** | **450** |
| 16 | 50 | 25 | microscrystals | 550 | 1 | 13 | - | - | - | **6.03** | **7200** | **800** |
| 17 | 50 | 18 | amorphous | 550 | 1 | **30** | **76** | 45 | 20 | **1.53** | **55000** | **1840** |
| 18 | 50 | 11 | amorphous | 550 | 1 | 48 | 93 | 73 | 36 | 0.95 | 113000 | 2650 |
| 19 | 50 | 4 | amorphous | 550 | 1 | 66 | 95 | 84 | 37 | 0.89 | 110000 | 2680 |
| 20 | 50 | Ar filling | amorphous | 550 | 1 | 67 | 95 | 84 | 36 | 0.86 | 114000 | 2590 |
| 21 | 50 | vacuum | amorphous | 550 | 1 | 67 | 96 | 82 | 35 | 0.80 | 115000 | 2810 |
| 22 | 30 | 25 | amorphous | 550 | 1 | **8** | - | - | - | **1.73** | **64000** | **2210** |
| 23 | 30 | 11 | amorphous | 550 | 1 | **13** | - | - | - | **1.83** | **54000** | **2100** |
| 24 | 30 | 4 | amorphous | 550 | 1 | **15** | - | - | - | **1.65** | **70000** | **2200** |
| 25 | 30 | Ar filling | amorphous | 550 | 1 | **13** | - | - | - | **1.67** | **55000** | **2100** |
| 26 | 30 | vacuum | amorphous | 550 | 1 | **14** | - | - | - | **1.59** | 63000 | **2000** |

Table 1 shows that there is a correlation between manufacturing conditions (roll temperature, vapor pressure in chamber, existence of crystals before heat treatment, and heat treatment conditions) and network structures (number of maximum points, coordination number, and volume ratio of network phase). Table 1 also shows that there is a correlation between network structure and magnetic properties of samples.

That is, Table 1 shows that amorphous ribbons are obtained under manufacturing conditions whose roll temperature was 50 to 70°C, vapor pressure was controlled to 11 or less hPa in a chamber of 30°C, and heat conditions were determined as 500 to 600°C and 0.5 to 10 hours. Then, it was confirmed that a favorable Fe network can be formed by carrying out a heat treatment against the ribbons. It was also confirmed that when a favorable Fe network can be formed, the sample has a decreased coercivity and an improved permeability.

On the other hand, the number of maximum points to be a condition of a preferable Fe network phase after a heat treatment tends to be small when a roll temperature is 30°C (Sample No. 22 to Sample 26) or when a roll temperature is 50°C or 70°C and a vapor pressure is higher than 11 hPa (Sample No. 1, Sample No. 12, Sample No. 16, and Sample No. 17). That is, it turned out that when a roll temperature is too low and a vapor pressure is too high at the time of manufacture of the ribbons, there is a tendency that the number of maximum points after a heat treatment is small after the ribbons are subjected to a heat treatment, and a favorable Fe network cannot be formed.

It also turned out that a favorable Fe network is not formed when a heat treatment temperature is too low (Sample No. 11) and a heat treatment time is too short (Sample No. 7). Then, it turned out that coercivity is high and permeability is low in these cases. The number of maximum points of Fe tended to decrease when a heat treatment is high (Sample No. 15) and a heat treatment time is too long (Sample No. 10).

It turned out that Sample No. 15 has a tendency that when a heat treatment temperature is high, coercivity deteriorates rapidly, and permeability decreases rapidly. It is conceived that this is because a part of the soft magnetic alloy forms boride (Fe₂B). The formation of boride in Sample No. 15 was confirmed using an X-ray diffraction measurement.

It turned out that magnetic properties or so of a sample can be anticipated by analyzing network structures of predetermined elements in the sample. It also turned out that the analysis of the network structure of specific elements in a sample can determine optimal manufacture conditions and develop a product having optimal characteristics. It also turned out that a correlation between a degree of network formation and characteristics (e.g., magnetic properties) of a sample can be analyzed in more detail by digitizing the degree of network formation and analyzing it.

### (Experiment 2)

An analysis was carried out in the same manner as Experiment 1 by changing a composition of a base alloy at a roll temperature of 70°C and a vapor pressure of 4 hPa in a chamber. Each sample was subjected to a heat treatment at 450°C, 500°C, 550°C, 600°C, and 650°C, and a temperature when coercivity was the lowest was determined as a heat treatment temperature.

Table 2 and Table 3 show characteristics at the temperature when coercivity was the lowest. That is, the samples had different heat treatment temperatures. Table 2 shows the results of an experiment carried out with Fe-Si-M-B-Cu-C based compositions. Table 3 shows the results of an experiment carried out with Fe-M-B-C based compositions.

Incidentally, Sample No. 39 was observed using a 3DAP with 5 nm thickness. FIG. 1 shows the results. FIG. 1 shows that a part having a high Fe content is distributed in network in the example of Sample No. 39.

**Table 2**

| Sample No. | Composition | Existence of crystals before heat treatment | Network structures | | | | Coercivity (A/m) | *µ*r (1kHz) | *µ*r (1 MHz) |
|---|---|---|---|---|---|---|---|---|---|
| | | | Number of maximum points (ten thousand / *µ* m³) | Coordination number is 1 or more and 5 or less (%) | Coordination number is 2 or more and 4 or less (%) | Fe network composition phase (vol%) | | | |
| 27 | Fe77.5Cu1Nb3Si13.5B5 | microscrystals | **11** | - | - | - | **9** | **5400** | **640** |
| 28 | Fe75.5Cu1Nb3Si13.5B7 | amorphous | 74 | 93 | 77 | 45 | 1.17 | 93000 | 2560 |
| 29 | Fe73.5Cu1Nb3Si13.5B9 | amorphous | 67 | 95 | 84 | 36 | 0.85 | 118000 | 2800 |
| 30 | Fe71.5Cu1Nb3Si13.5B11 | amorphous | 58 | 90 | 76 | 32 | 0.84 | 103000 | 2620 |
| 31 | Fe69.5Cu1Nb3Si13.5B13 | amorphous | 52 | 85 | 72 | 33 | 0.94 | 97000 | 2540 |
| 32 | Fe74.5Nb3Si13.5B9 | microscrystals | **7** | - | - | - | **14** | **3500** | **400** |
| 33 | Fe74.4Cu0.1Nb3Si13.5B9 | amorphous | 41 | 81 | 63 | 25 | 1.33 | 55000 | 2550 |
| 34 | Fe73.5Cu1Nb3Si13.5B9 | amorphous | 67 | 95 | 84 | 36 | 0.85 | 118000 | 2800 |
| 35 | Fe71.5Cu3Nb3Si13.5B9 | amorphous | 62 | 95 | 69 | 33 | 1.17 | 75000 | 2320 |
| 36 | Fe71 Cu3.5Nb3Si13.5B9 | crystals | **No ribbon is manufactured** | | | | | | |
| 37 | Fe79.5Cu1Nb3Si9.5B9 | microscrystals | **7** | - | - | - | **24** | **2000** | **440** |
| 38 | Fe75.5Cu1Nb3Si11.5B9 | amorphous | 71 | 87 | 69 | 34 | 1.04 | 92000 | 2450 |
| 39 | Fe73.5Cu1Nb3Si13.5B9 | amorphous | 67 | 95 | 84 | 36 | 0.85 | 118000 | 2800 |
| 40 | Fe73.5Cu1Nb3Si15.5B7 | amorphous | 63 | 95 | 80 | 36 | 0.78 | 118000 | 2840 |
| 41 | Fe71.5Cu1Nb3Si15.5B9 | amorphous | 60 | 94 | 83 | 40 | 0.79 | 120000 | 2730 |
| 42 | Fe69.5Cu1Nb3Si17.5B9 | amorphous | 54 | 93 | 81 | 49 | 0.89 | 100200 | 2360 |
| 43 | Fe76.5Cu1Si13.5B9 | crystals | - | - | - | - | **2800** | **1500** | **250** |
| 44 | Fe75.5Cu1Nb1Si13.5B9 | amorphous | 45 | 85 | 67 | 24 | 1.32 | 73000 | 2540 |
| 45 | Fe73.5Cu1Nb3Si13.5B9 | amorphous | 67 | 95 | 84 | 36 | 0.85 | 118000 | 2800 |
| 46 | Fe71.5Cu1Nb5Si13.5B9 | amorphous | 63 | 92 | 82 | 34 | 0.95 | 110000 | 2740 |
| 47 | Fe66.5Cu1Nb10Si13.5B9 | amorphous | 58 | 91 | 72 | 38 | 1.03 | 98000 | 2600 |
| 48 | Fe73.5Cu1Ti3Si13.5B9 | amorphous | 64 | 85 | 61 | 31 | 1.39 | 51000 | 2320 |
| 49 | Fe73.5Cu1 Zr3Si13.5B9 | amorphous | 65 | 83 | 63 | 27 | 1.45 | 53000 | 2310 |
| 50 | Fe73.5Cu1Hf3Si13.5B9 | amorphous | 68 | 82 | 64 | 29 | 1.4 | 54000 | 2350 |
| 51 | Fe73.5Cu1V3Si13.5B9 | amorphous | 67 | 84 | 68 | 29 | 1.32 | 55000 | 2250 |
| 52 | Fe73.5Cu1 Ta3Si13.5B9 | amorphous | 67 | 81 | 62 | 25 | 1.52 | 50000 | 2320 |
| 53 | Fe73.5Cu1Mo3Si13.5B9 | amorphous | 58 | 85 | 68 | 23 | 1.32 | 68000 | 2480 |
| 54 | Fe73.5Cu1Hf1.5N61.5Si13.5B9 | amorphous | 71 | 93 | 77 | 34 | 1.34 | 78000 | 2640 |
| 55 | Fe79.5Cu1Nb2Si9.5B9C1 | amorphous | 43 | 82 | 55 | 22 | 1.47 | 52000 | 2350 |
| 56 | Fe79Cu1Nb2Si9B5C4 | amorphous | 48 | 81 | 62 | 25 | 1.43 | 56000 | 2270 |
| 57 | Fe73.5Cu1Nb3Si13.5B8C1 | amorphous | 66 | 95 | 84 | 37 | 0.77 | 121000 | 2830 |
| 58 | Fe73.5Cu1Nb3Si13.5B5C4 | amorphous | 54 | 90 | 77 | 33 | 1.01 | 98000 | 2550 |
| 59 | Fe69.5Cu1Nb3Si17.5B8C1 | amorphous | 42 | 81 | 63 | 33 | 1.21 | 89000 | 2460 |
| 60 | Fe69.5Cu1Nb3Si17.5B5C4 | amorphous | 44 | 82 | 58 | 35 | 1.31 | 71000 | 2300 |

**Table 3**

| Sample No. | Composition | State before heat treatment (amorphous or crystals) | Network structures | | | | Coercivity (A/m) | *µ*r (1kHz) | *µ*r (1MHz) |
|---|---|---|---|---|---|---|---|---|---|
| | | | Number of maximum points (ten thousand /*µ* m³) | Coordination number is 1 or more and 5 or less (%) | Coordination number is 2 or more and 4 or less (%) | Fe network composition phase (vol%) | | | |
| 61 | Fe88Nb3B9 | crystals | - | - | - | - | **15000** | **900** | **300** |
| 62 | Fe86Nb5B9 | amorphous | 82 | 89 | 70 | 38 | 12.3 | 25000 | 1800 |
| 63 | Fe84Nb7B9 | amorphous | 107 | 93 | 83 | 37 | 5.5 | 43000 | **2200** |
| 64 | Fe81Nb10B9 | amorphous | 120 | 94 | 84 | 39 | 5.4 | 52000 | 2150 |
| 65 | Fe77Nb14B9 | amorphous | 115 | 91 | 82 | 36 | 4.8 | 55000 | 2180 |
| 66 | Fe90Nb7B3 | crystals | - | - | - | - | **20000** | **2100** | **600** |
| 67 | Fe87Nb7B6 | amorphous | 89 | 81 | 67 | 29 | 9.5 | 35000 | 1600 |
| 68 | Fe84Nb7B9 | amorphous | 107 | 93 | 83 | 37 | 5.5 | 43000 | 2200 |
| 69 | Fe81Nb7B12 | amorphous | 93 | 91 | 75 | 34 | 4.9 | 45000 | 2100 |
| 70 | Fe75Nb7B18 | amorphous | 86 | 93 | 76 | 31 | 3.9 | 58000 | 1930 |
| 71 | Fe84Nb7B9 | amorphous | 107 | 93 | 83 | 37 | 5.5 | 43000 | 2100 |
| 72 | Fe83.9Cu0.1Nb7B9 | amorphous | 121 | 90 | 84 | 36 | 3.9 | 59000 | 2200 |
| 73 | Fe83Cu2Nb7B9 | amorphous | 141 | 91 | 87 | 39 | 3.7 | 60000 | 2350 |
| 74 | Fe81Cu3Nb7B9 | crystals | - | - | - | - | **18000** | **2100** | **650** |
| 75 | Fe85.9Cu0.1Nb5B9 | microscrvstals | **30** | - | - | - | **25** | **10000** | 1300 |
| 76 | Fe83.9Cu0.1Nb7B9 | amorphous | 121 | 90 | 84 | 36 | 3.9 | 59000 | 2200 |
| 77 | Fe80.9Cu0.1Nb10B9 | amorphous | 130 | 88 | 83 | 39 | 3.7 | 65000 | 1800 |
| 78 | Fe76.9Cu0.1Nb14B9 | amorphous | 106 | 86 | 65 | 47 | 4.8 | 37000 | 1840 |
| 79 | Fe89.9Cu0.1Nb7B3 | microscrvstals | **35** | - | - | - | **16000** | **1800** | **560** |
| 80 | Fe88.4Cu0.1Nb7B4.5 | amorphous | 138 | 95 | 86 | 36 | 9.9 | 48000 | 1950 |
| 81 | Fe83.9Cu0.1Nb7B9 | amorphous | 121 | 90 | 84 | 36 | 3.9 | 59000 | 2200 |
| 82 | Fe809Cu0.1Nb7B12 | amorphous | 110 | 85 | 76 | 32 | 6.3 | 38000 | 1930 |
| 83 | Fe749Cu0.1Nb7B18 | amorphous | 98 | 81 | 69 | 45 | 7.8 | 25000 | 1880 |
| 84 | Fe91Zr7B2 | amorphous | 83 | 94 | 82 | 37 | 6.8 | 23000 | 1500 |
| 85 | Fe90Zr7B3 | amorphous | 92 | 97 | 89 | 35 | 3.7 | 42000 | 1890 |
| 86 | Fe89Zr7B3Cu1 | amorphous | 110 | 93 | 83 | 36 | 4.1 | 49000 | 2010 |
| 87 | Fe90Hf7B3 | amorphous | 109 | 93 | 83 | 36 | 5.1 | 38000 | 1840 |
| 88 | Fe89Hf7B4 | amorphous | 111 | 91 | 88 | 35 | 3.9 | 45000 | 1930 |
| 89 | Fe88Hf7B3Cu1 | amorphous | 133 | 90 | 73 | 38 | 2.7 | 60000 | 2160 |
| 90 | Fe84Nb3.5Zr3.5B8Cu1 | amorphous | 125 | 93 | 87 | 35 | 1.4 | 110000 | 2790 |
| 91 | Fe84Nb3.5Hf3.5B8Cu1 | amorphous | 125 | 94 | 88 | 35 | 1.1 | 100000 | 2570 |
| 92 | Fe90.9Nb6B3C0.1 | amorphous | 89 | 81 | 67 | 36 | 5.9 | 24000 | 1300 |
| 93 | Fe93.06Nb2.97B2.97C1 | amorphous | 67 | 89 | 78 | 37 | 4.8 | 30000 | 1600 |
| 94 | Fe94.05Nb1.98B2.97C1 | amorphous | 54 | 85 | 74 | 37 | 4.9 | 56000 | 2100 |
| 95 | Fe909Nb1.98B297C4 | amorphous | 46 | 93 | 85 | 35 | 3.1 | 64000 | 2300 |
| 96 | Fe90.9Nb3B6C0.1 | amorphous | 77 | 93 | 77 | 34 | 5.8 | 28000 | 1400 |
| 97 | Fe94.5Nb3B2C0.5 | amorphous | 65 | 93 | 82 | 38 | 4.8 | 23000 | 1380 |
| 98 | Fe83.9Nb7B9C0.1 | amorphous | 121 | 92 | 79 | 39 | 3.6 | 42000 | 1860 |
| 99 | Fe80.8Nb6.7B8.65C3.85 | amorphous | 132 | 97 | 89 | 40 | 2.8 | 79000 | 2300 |
| 100 | Fe77.9Nb14B8C0.1 | amorphous | 98 | 83 | 64 | 32 | 7.6 | 23000 | 1700 |
| 101 | Fe75Nb13.5B7.5C4 | amorphous | 76 | 94 | 84 | 39 | 3.2 | 64000 | 2130 |
| 102 | Fe78Nb1B17C4 | amorphous | 56 | 93 | 72 | 41 | 11.2 | 34000 | 1400 |
| 103 | Fe78Nb1B20C1 | amorphous | 64 | 90 | 77 | 44 | 10.3 | 23000 | 1390 |

As shown in Table 2 and Table 3, a ribbon obtained by a single roll method at a roll temperature of 70°C and a vapor pressure of 4 hPa can form amorphous even if a base alloy has different compositions, and a heat treatment at an appropriate temperature forms a preferable Fe composition network topology structure, decreases coercivity, and improves permeability.

Samples having a Fe-Si-M-B-Cu-C based composition and a network structure shown in Table 2 tend to have a relatively small number of maximum points, and samples having a Fe-M-B-C based composition and a network structure shown in Table 3 tend to have a relatively large number of maximum points.

In samples having a Fe-Si-M-B-Cu-C based composition shown in Table 2, particularly Sample No. 32 to Sample No. 36, the number of maximum points of Fe tends to increase by a small amount of addition of Cu. When a Cu content is too large, there is a tendency that a ribbon before a heat treatment obtained by a single roll method contains crystals, and a favorable Fe network are not formed.

In samples having a Fe-Si-M-B-Cu-C based composition shown in Table 2, particularly Sample No. 43 to Sample No. 47, a sample having a smaller amount of Nb shows that a ribbon obtained by a single roll method tends to easily contain crystals. A sample having a larger amount of Nb tends to easily have a decreased number of maximum points of Fe and a decreased permeability.

In samples having a Fe-Si-M-B-Cu-C based composition shown in Table 2, particularly Sample No. 27 to Sample No. 31, a sample having a smaller amount of B shows that a ribbon before a heat treatment obtained by a single roll method tends to easily contain microcrystals. A sample having a larger amount of B tends to easily have a decreased number of maximum points of Fe and a decreased permeability.

In samples having a Fe-Si-M-B-Cu-C based composition shown in Table 2, particularly Sample No. 37 to Sample No. 42, a sample having a smaller amount of Si tends to have a decreased permeability.

Samples having a Fe-Si-M-B-Cu-C based composition shown in Table 2, particularly Sample No. 55 and Sample No. 56, tend to maintain amorphous even in a range having an increased amount of Fe by containing C and form a favorable Fe network.

In samples having a Fe-M-B-C based composition shown in Table 3, particularly Sample No. 61 to Sample No. 65, a sample having a smaller amount of M shows that a ribbon before a heat treatment obtained by a single roll method tends to contain crystals.

In samples having a Fe-M-B-C based composition shown in Table 3, particularly Sample No. 66 to Sample No. 70, a sample having a smaller amount of B shows that a ribbon before a heat treatment obtained by a single roll method tends to contain crystals, and a sample having a larger amount of B shows that the number of maximum points of Fe tends to decrease.

As a result of similar examination with respect to Sample No. 71 to Sample No. 103 in Table 3, it was confirmed that amorphous was formed in a soft magnetic alloy ribbon having an appropriate composition and manufactured with a roll temperature of 70°C and a vapor pressure of 4 hPa in a chamber. Then, the samples tend to have a network structure of Fe, a low coercivity, and a high permeability by carrying out an appropriate heat treatment.

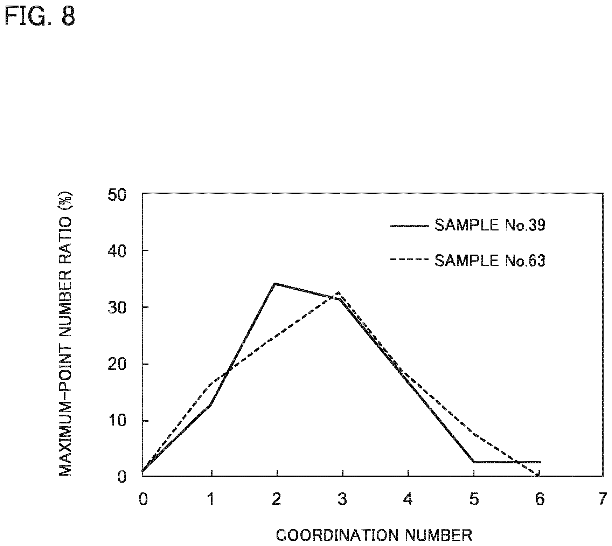

A coordination number distribution of all maximum points with respect to Sample No. 39 of Table 2 and Sample No. 63 of Table 3 was graphed. FIG. 8 shows the graphed results. In FIG. 8, a horizontal axis represents a coordination number, and a vertical axis represents a maximum-point number ratio taking the coordination number. The total number of maximum points is 100%, and the vertical axis represents a ratio of maximum points taking each coordination number.

FIG. 8 shows that the Fe-Si-M-B-Cu-C based composition shown in Table 2 has a smaller variation of coordination number than that of the Fe-M-B-C based composition shown in Table 3.

### (Experiment 3)

Each of pure metal materials was weighed so that a base alloy having a composition of Fe: 73.5 atom%, Si: 13.5 atom%, B: 9.0 atom%, Nb: 3.0 atom%, and Cu: 1.0 atom% was obtained. Then, the base alloy was manufactured by evacuating a chamber and thereafter melting the pure metal materials at high frequencies.

Then, the manufactured base alloy was heated and molten to be turned into a metal in a molten state at 1300°C. This metal was thereafter sprayed by a gas atomizing method in predetermined conditions shown in Table 4 below, and powders were manufactured. In Experiment 3, Sample No. 104 to Sample No. 107 were manufactured by changing a gas spray temperature and a vapor pressure in a chamber. The vapor pressure was adjusted using an Ar gas whose dew point had been adjusted.

Each of the powders before the heat treatment was subjected to an X-ray diffraction measurement for confirmation of existence of crystals. In addition, a restricted visual field diffraction image and a bright field image were observed by a transmission electron microscope. As a result, it was confirmed that each powder had no crystals and was complete amorphous.

Then, each of the obtained powders was subjected to a heat treatment and thereafter measured with respect to coercivity. Then, a Fe composition network was analyzed. A heat treatment temperature of samples having a Fe-Si-M-B-Cu-C based composition was 550°C, and a heat treatment temperature of samples having a Fe-M-B-C based composition was 600°C. The heat treatment was carried out for 1 hour.

**Table 4**

| Sample No. | Composition | Gas temperature (°C) | Vapor pressure (hPa) | Network structures | | | | Coercivity (A/m) |
|---|---|---|---|---|---|---|---|---|
| | | | | Number of maximum points (ten thousand /*µ*m³) | Coordination number is 1 or more and 5 or less (%) | Coordination number is 2 or more and 4 or less (%) | Fe network composition phase (vol%) | |
| 104 | Fe73.5Cu1Nb3Si13.5B9 | 30 | 25 | **13** | - | - | - | **38** |
| 105 | Fe73.5Cu1Nb3Si13.5B9 | 100 | 4 | 67 | 93 | 84 | 35 | 24 |
| 106 | Fe84Nb7B9 | 30 | 25 | **32** | - | - | - | **280** |
| 107 | Fe84Nb7B9 | 100 | 4 | 109 | 94 | 84 | 36 | 98 |

In Sample No. 105 and Sample No. 107, a favorable Fe network was formed by appropriately carrying out a heat treatment against the complete amorphous powders. Comparative examples of Sample No. 104 and Sample No. 106, which have a too low gas temperature of 30°C and a too high vapor pressure of 25 hPa, however, had a small number of maximum points after a heat treatment, no favorable Fe composition network, and a high coercivity.

When comparing comparative examples and examples shown in Table 4, it was found that an amorphous soft magnetic alloy powder was obtained by changing a gas spray temperature, and that the number of maximum points of Fe increased and a Fe composition network structure was obtained in the same manner as a ribbon by carrying out a heat treatment against the amorphous soft magnetic alloy powder. In addition, coercivity tends to be small by having a Fe network structure in the same manner as the ribbons of Experiments 1 to 3.

### (Experiment 4: Sample No. 201 to Sample No. 226)

Single-plate samples were obtained in the same manner as Experiment 1. Each sample shown in Table 5 was manufactured in the same manner as Experiment 1 by changing a roll temperature, a vapor pressure, and heat treatment conditions. Then, each sample after each ribbon was subjected to a heat treatment was measured in the same manner as Experiment 1 with respect to coercivity, permeability at 1 kHz frequency, and permeability at 1 MHz frequency of each sample after each ribbon was subjected to a heat treatment. Table 5 shows the results.

Moreover, each sample was measured with respect to Fe content using a three-dimensional atom probe (3DAP), and a virtual-line total distance, a virtual-line average distance, and a virtual-line standard deviation were analyzed based on the results using a program of the analysis method shown in Second Embodiment of the present invention. In addition, an existence ratio of virtual lines having a length of 4 to 16 nm and a volume ratio of a Fe network composition phase were analyzed. Table 5 shows the results.

Incidentally, samples expressing "< 1" in columns of virtual-line total distance are samples having no virtual lines between a Fe maximum point and a Fe maximum point. When a Fe maximum point and a Fe maximum point are adjacent to each other, however, an extremely short virtual line may be considered to exist between the two adjacent Fe maximum points at the time of calculation of virtual-line total distance. In this case, the virtual-line total distance may be considered to be 0.0001 mm/µm³. In the present application, "< 1" is thus written in the columns of virtual-line total distance as a description including a virtual-line total distance of 0 mm/µm³ and a virtual-line total distance of 0.0001 mm/µm³. Incidentally, such an extremely short virtual line is considered to fail to exist at the time of calculation of virtual-line average distance and/or virtual-line standard deviation.

**Table 5**

| Sample No. | Roll temperature (°C) | Vapor pressure in chamber (hPa) | Existence of crystals before heat treatment | Heat treatment conditions | | Network structures | | | | | Coercivity (A/m) | *µ*r (1kHz) | *µ*r (1MHz) |
|---|---|---|---|---|---|---|---|---|---|---|---|---|---|
| | | | | Heat treatment temperature (°C) | Heat treatment time (h) | Virtual-line total distance (mm/µm³) | Virtual-line average distance (nm) | Virtual-line standard deviation (nm) | Existence ratio of 4 to 16 nm virtual lines (%) | Fe network composition phase (vol%) | | | |
| 201 | 70 | 25 | microscrystals | 550 | 1 | <1 | - | - | - | - | **7.03** | **6200** | **730** |
| 202 | 70 | 18 | amorphous | 550 | 1 | <1 | - | - | - | - | **1.86** | **63000** | **1900** |
| 203 | 70 | 11 | amorphous | 550 | 1 | 11 | 8 | 3.6 | 88 | 35 | 0.96 | 103000 | 2700 |
| 204 | 70 | 4 | amorphous | 550 | 1 | 14 | 9 | 3.6 | 91 | 36 | 0.85 | 118000 | 2800 |
| 205 | 70 | Ar filling | amorphous | 550 | 1 | 13 | 9 | 3.8 | 89 | 36 | 0.79 | 110000 | 2670 |
| 206 | 70 | vacuum | amorphous | 550 | 1 | 15 | 8 | 3.4 | 91 | 35 | 0.73 | 108000 | 2560 |
| 207 | 70 | 4 | amorphous | 550 | 0.1 | **7** | 6 | 3.4 | 77 | 18 | **1.23** | **52000** | **1800** |
| 208 | 70 | 4 | amorphous | 550 | 0.5 | 13 | 7 | 3.2 | 85 | 31 | 0.82 | 108000 | 2730 |
| 209 | 70 | 4 | amorphous | 550 | 10 | 12 | 10 | 3.8 | 91 | 41 | 0.92 | 103000 | 2570 |
| 210 | 70 | 4 | amorphous | 550 | 100 | **2** | **5** | 2.9 | 55 | 54 | 1.25 | **68000** | 1800 |
| 211 | 70 | 4 | amorphous | 450 | 1 | <1 | - | - | - | - | 1.40 | **40000** | 1500 |
| 212 | 70 | 4 | amorphous | 500 | 1 | 12 | 7 | 3.2 | 82 | 31 | 0.82 | 108000 | 2730 |
| 213 | 70 | 4 | amorphous | 550 | 1 | 14 | 9 | 4 | 85 | 37 | 0.86 | 107000 | 2580 |
| 214 | 70 | 4 | amorphous | 600 | 1 | 12 | 11 | 4.6 | 88 | 41 | 0.94 | 101000 | 2570 |
| 215 | 70 | 4 | amorphous | 650 | 1 | 15 | **13** | 7.1 | 75 | 52 | **48** | **2000** | **450** |
| 216 | 50 | 25 | microscrystals | 550 | 1 | <1 | - | - | - | - | **6.03** | **7200** | **800** |
| 217 | 50 | 18 | amorphous | 550 | 1 | **4** | **4** | 2.5 | 40 | 20 | **1.53** | **55000** | **1840** |
| 218 | 50 | 11 | amorphous | 550 | 1 | 10 | 10 | 4.1 | 88 | 36 | 0.95 | 113000 | 2650 |
| 219 | 50 | 4 | amorphous | 550 | 1 | 14 | 8 | 3.4 | 90 | 37 | 0.89 | 110000 | 2680 |
| 220 | 50 | Ar filling | amorphous | 550 | 1 | 13 | 8 | 3.3 | 92 | 36 | 0.86 | 114000 | 2590 |
| 221 | 50 | vacuum | amorphous | 550 | 1 | 14 | 9 | 3.8 | 90 | 35 | 0.80 | 115000 | 2810 |
| 222 | 30 | 25 | amorphous | 550 | 1 | <1 | - | - | - | - | **1.73** | **64000** | **2210** |
| 223 | 30 | 11 | amorphous | 550 | 1 | <1 | - | - | - | - | **1.83** | **54000** | **2100** |
| 224 | 30 | 4 | amorphous | 550 | 1 | <1 | - | - | - | - | **1.65** | **70000** | **2200** |
| 225 | 30 | Ar filling | amorphous | 550 | 1 | <1 | - | - | - | - | **1.67** | **55000** | **2100** |
| 226 | 30 | vacuum | amorphous | 550 | 1 | <1 | - | - | - | - | **1.59** | **630000** | **2000** |

Table 5 shows that an amorphous ribbon was obtained in samples where a roll temperature was 50 to 70°C, a vapor pressure was controlled to 11 or less hPa in a chamber of 30°C, and heat treatment conditions were 500 to 600°C and 0.5 to 10 hours. A favorable Fe network was formed by carrying out a heat treatment against the ribbon. Then, coercivity decreased, and permeability improved.

On the other hand, when a roll temperature was 30°C (Sample No. 222 to Sample No. 226) or when a roll temperature was 50°C or 70°C and a vapor pressure was higher than 11 hPa (Sample No. 201, Sample No. 202, Sample No. 216, and Sample No. 217), there was a tendency that a virtual-line total distance and a virtual-line average distance to be conditions of a favorable Fe network phase after a heat treatment were out of predetermined ranges, or that no virtual lines were observed. That is, when a roll temperature was too low and a vapor pressure was too high at the time of manufacture of the ribbon, a favorable Fe network could not be formed after a heat treatment of the ribbon.

When a heat treatment temperature was too low (Sample No. 211) and a heat treatment time was too short (Sample No. 207), a favorable Fe network was not formed. Then, when no Fe network was formed, coercivity was high, and permeability was low. When a heat treatment temperature was high (Sample No. 215) and a heat treatment time was too long (Sample No. 210), the number of maximum points of Fe tended to decrease. In Sample No 215, when a heat treatment temperature was high, there was a tendency that coercivity deteriorated rapidly and permeability decreased rapidly. It is conceived that this is because a part of the soft magnetic alloy formed boride (Fe₂B). The formation of boride in Sample No. 215 was confirmed using an X-ray diffraction measurement.

It turned out that magnetic properties or so of a sample can be anticipated by analyzing network structures of predetermined elements in the sample. It also turned out that the analysis of the network structure of specific elements in a sample can determine optimal manufacture conditions and develop a product having optimal characteristics. It also turned out that a correlation between a degree of network formation and characteristics (e.g., magnetic properties) of a sample can be analyzed in more detail by digitizing the degree of network formation and analyzing it.

### (Experiment 5)

Samples having a Fe-Si-M-B-Cu-C based composition were prepared in the same manner as Experiment 2 and analyzed with respect to a virtual-line total distance, a virtual-line average distance, and a virtual-line standard deviation in the same manner as Experiment 4. Moreover, an existence ratio of virtual lines having a length of 4 to 16 nm and a volume ratio of a Fe network composition phase were analyzed. Table 6 shows the results. Table 7 shows the results analyzed by a Fe-M-B-C based composition.

**Table 6**

| Sample No. | Composition | Existence of crystals before heat treatment | Network structures | | | | | Coercivity (A/m) | *µ*r (1 kHz) | *µ*r (1 MHz) |
|---|---|---|---|---|---|---|---|---|---|---|
| | | | Virtual-line total distance (mm/µm³) | Virtual-line average distance (nm) | Virtual-line standard deviation (nm) | Existence ratio of 4 to 16 nm virtual lines (%) | Fe network composition phase (vol%) | | | |
| 227 | Fe77.5Cu1 Nb3Si13.5B5 | microscrystals | **<1** | - | - | - | - | **9** | **5400** | **640** |
| 228 | Fe75.5Cu1Nb3Si13.5B7 | amorphous | 17 | 7 | 3.1 | 87 | 45 | 1.17 | 93000 | 2560 |
| 229 | Fe73.5Cu1Nb3Si13.5B9 | amorphous | 14 | 9 | 3.6 | 90 | 36 | 0.85 | 118000 | 2800 |
| 230 | Fe71.5Cu1Nb3Si13.5B11 | amorphous | 12 | 7 | 3.0 | 91 | 32 | 0.84 | 103000 | 2620 |
| 231 | Fe69.5Cu1Nb3Si13.5B13 | amorphous | 11 | 6 | 3.2 | 84 | 33 | 0.94 | 97000 | 2540 |
| 232 | Fe74.5Nb3Si13.5B9 | microscrystals | **<1** | **-** | **-** | **-** | **-** | **14** | **3500** | **400** |
| 233 | Fe74.4Cu0.1Nb3Si13.5B9 | amorphous | 10 | 6 | 3.6 | 82 | 25 | 1.33 | 55000 | 2550 |
| 234 | Fe73.5Cu1Nb3Si13.5B9 | amorphous | 13 | 10 | 4.2 | 87 | 36 | 0.85 | 118000 | 2800 |
| 235 | Fe71.5Cu3Nb3Si13.5B9 | amorphous | 12 | 9 | 3.9 | 89 | 33 | 1.17 | 75000 | 2320 |
| 236 | Fe71 Cu3.5Nb3Si13.5B9 | crystals | **No ribbon is manufactured.** | | | | | | | |
| 237 | Fe79.5Cu1Nb3Si9.5B9 | microscrystals | **<1** | **-** | **-** | **-** | **-** | **24** | **2000** | **440** |
| 238 | Fe75.5Cu1Nb3Si11.5B9 | amorphous | 16 | 7 | 3.6 | 83 | 34 | 1.04 | 92000 | 2450 |
| 239 | Fe73.5Cu1Nb3Si13.5B9 | amorphous | 14 | 8 | 3.9 | 85 | 36 | 0.85 | 118000 | 2800 |
| 240 | Fe73.5Cu1Nb3Si15.5B7 | amorphous | 13 | 8 | 3.7 | 88 | 36 | 0.78 | 118000 | 2840 |
| 241 | Fe71.5Cu1Nb3Si15.5B9 | amorphous | 13 | 10 | 4.2 | 87 | 40 | 0.79 | 120000 | 2730 |
| 242 | Fe69.5Cu1Nb3Si17.5B9 | amorphous | 11 | 12 | 5.1 | 82 | 49 | 0.89 | 100200 | 2360 |
| 243 | Fe76.5Cu1Si13.5B9 | crystals | **<1** | **-** | **-** | **-** | **-** | **2800** | **1500** | **250** |
| 244 | Fe75.5Cu1Nb1 Si13.5B9 | amorphous | 10 | 6 | 3.7 | 82 | 24 | 1.32 | 73000 | 2540 |
| 245 | Fe73.5Cu1Nb3Si13.5B9 | amorphous | 13 | 9 | 4.0 | 88 | 36 | 0.85 | 118000 | 2800 |
| 246 | Fe71.5Cu1Nb5Si13.5B9 | amorphous | 14 | 8 | 3.6 | 90 | 34 | 0.95 | 110000 | 2740 |
| 247 | Fe66.5Cu1Nb10Si13.5B9 | amorphous | 11 | 8 | 4.0 | 84 | 38 | 1.03 | 98000 | 2600 |
| 248 | Fe73.5Cu1Ti3Si13.5B9 | amorphous | 13 | 7 | 3.3 | 86 | 31 | 1.39 | 51000 | 2320 |
| 249 | Fe73.5Cu1Zr3Si13.5B9 | amorphous | 10 | 7 | 3.3 | 88 | 27 | 1.45 | 53000 | 2310 |
| 250 | Fe73.5Cu1Hf3Si13.5B9 | amorphous | 11 | 7 | 3.4 | 88 | 29 | 1.4 | 54000 | 2350 |
| 251 | Fe73.5Cu1V3Si13.5B9 | amorphous | 12 | 7 | 3.3 | 88 | 29 | 1.32 | 55000 | 2250 |
| 252 | Fe73.5Cu1Ta3Si13.5B9 | amorphous | 11 | 8 | 3.4 | 91 | 25 | 1.52 | 50000 | 2320 |
| 253 | Fe73.5Cu1Mo3Si13.5B9 | amorphous | 10 | 7 | 3.2 | 87 | 23 | 1.32 | 68000 | 2480 |
| 254 | Fe73.5Cu1Hf1.5Nb1.5Si13.5B9 | amorphous | 16 | 9 | 4.2 | 83 | 34 | 1.34 | 78000 | 2640 |
| 255 | Fe79.5Cu1Nb2Si9.5B9C1 | amorphous | 10 | 6 | 3.8 | 80 | 22 | 1.47 | 52000 | 2350 |
| 256 | Fe79Cu1 Nb2Si9B5C4 | amorphous | 10 | 6 | 3.7 | 81 | 25 | 1.43 | 56000 | 2270 |
| 257 | Fe73.5Cu1Nb3Si13.5B8C1 | amorphous | 13 | 9 | 4.1 | 87 | 37 | 0.77 | 121000 | 2830 |
| 258 | Fe73.5Cu1Nb3Si13.5B5C4 | amorphous | 12 | 7 | 3.0 | 91 | 33 | 1.01 | 98000 | 2550 |
| 259 | Fe69.5Cu1Nb3Si17.5B8C1 | amorphous | 11 | 6 | 3.7 | 81 | 33 | 1.21 | 89000 | 2460 |
| 260 | Fe69.5Cu1Nb3Si17.5B5C4 | amorphous | 12 | 6 | 3.7 | 81 | 35 | 1.31 | 71000 | 2300 |

**Table 7**

| Sample No. | Composition | State before heat treatment (amorphous or crystals) | Network structures | | | | | Coercivity (A/m) | *µ*r (1 KHz) | *µ*r (1 MHz) |
|---|---|---|---|---|---|---|---|---|---|---|
| | | | Virtual-line total distance (mm/µm³) | Virtual-line average distance (nm) | Virtual-line standard deviation (nm) | Existence ratio of 4 to 16 nm virtual lines (%) | Fe network composition phase (vol%) | | | |
| 261 | Fe88Nb3B9 | crystals | **<1** | - | - | - | - | **15000** | **900** | **300** |
| 262 | Fe86Nb5B9 | amorphous | 17 | 8 | 4.0 | 84 | 38 | 12.3 | 25000 | 1800 |
| 263 | Fe84Nb7B9 | amorphous | 20 | 8 | 3.4 | 92 | 37 | 5.5 | 43000 | 2200 |
| 264 | Fe81Nb10B9 | amorphous | 21 | 9 | 4.0 | 88 | 39 | 5.4 | 52000 | 2150 |
| 265 | Fe77Nb14B9 | amorphous | 21 | 9 | 4.2 | 86 | 36 | 4.8 | 55000 | 2180 |
| 266 | Fe90Nb7B3 | crystals | <1 | - | - | - | - | **20000** | **2100** | **600** |
| 267 | Fe87Nb7B6 | amorphous | 15 | 7 | 3.9 | 81 | 29 | 9.5 | 35000 | 1600 |
| 268 | Fe84Nb7B9 | amorphous | 20 | 7 | 3.3 | 90 | 37 | 5.5 | 43000 | 2200 |
| 269 | Fe81Nb7B12 | amorphous | 16 | 8 | 3.7 | 87 | 34 | 4.9 | 45000 | 2100 |
| 270 | Fe75Nb7B18 | amorphous | 16 | 9 | 4.2 | 85 | 31 | 3.9 | 58000 | 1930 |
| 271 | Fe84Nb7B9 | amorphous | 19 | 8 | 3.8 | 85 | 37 | 5.5 | 43000 | 2100 |
| 272 | Fe83.9Cu0.1Nb7B9 | amorphous | 21 | 6 | 2.8 | 84 | 36 | 3.9 | 59000 | 2200 |
| 273 | Fe83Cu2Nb7B9 | amorphous | 23 | 6 | 2.7 | 85 | 39 | 3.7 | 60000 | 2350 |
| 274 | Fe81Cu3Nb7B9 | crystals | <1 | - | - | - | - | **18000** | **2100** | **650** |
| 275 | Fe85.9Cu0.1Nb5B9 | microscrystals | **4** | **5** | 3.0 | 51 | - | 25 | **10000** | 1300 |
| 276 | Fe83.9Cu0.1Nb7B9 | amorphous | 22 | 7 | 3.6 | 83 | 36 | 3.9 | 59000 | 2200 |
| 277 | Fe80.9Cu0.1Nb10B9 | amorphous | 23 | 6 | 2.9 | 82 | 39 | 3.7 | 65000 | 1800 |
| 278 | Fe76.9Cu0.1Nb14B9 | amorphous | 25 | 7 | 4.0 | 80 | 47 | 4.8 | 37000 | 1840 |
| 279 | Fe89.9Cu0.1Nb7B3 | microscrystals | 6 | 6 | 3.9 | 67 | - | **16000** | **1800** | **560** |
| 280 | Fe88.4Cu0.1Nb7B4.5 | amorphous | 21 | 6 | 2.6 | 85 | 36 | 9.9 | 48000 | 1950 |
| 281 | Fe83.9Cu0.1Nb7B9 | amorphous | 20 | 7 | 3.5 | 87 | 36 | 3.9 | 59000 | 2200 |
| 282 | Fe80.9Cu0.1Nb7B12 | amorphous | 20 | 7 | 3.7 | 83 | 32 | 6.3 | 38000 | 1930 |
| 283 | Fe74.9Cu0.1Nb7B18 | amorphous | 24 | 6 | 3.0 | 81 | 45 | 7.8 | 25000 | 1880 |
| 284 | Fe91Zr7B2 | amorphous | 20 | 8 | 3.5 | 88 | 37 | 6.8 | 23000 | 1500 |
| 285 | Fe90Zr7B3 | amorphous | 19 | 8 | 3.1 | 94 | 35 | 3.7 | 42000 | 1890 |
| 286 | Fe89Zr7B3Cu1 | amorphous | 19 | 7 | 3.4 | 89 | 36 | 4.1 | 49000 | 2010 |
| 287 | Fe90Hf7B3 | amorphous | 20 | 7 | 3.5 | 86 | 36 | 5.1 | 38000 | 1840 |
| 288 | Fe89Hf7B4 | amorphous | 19 | 8 | 3.3 | 90 | 35 | 3.9 | 45000 | 1930 |
| 289 | Fe88Hf7B3Cu1 | amorphous | 21 | 6 | 2.9 | 83 | 38 | 2.7 | 60000 | 2160 |
| 290 | Fe84Nb3.5Zr35B8Cu1 | amorphous | 20 | 7 | 3.5 | 85 | 35 | 1.4 | 110000 | 2790 |
| 291 | Fe84Nb3.5Hf3.5B8Cu1 | amorphous | 20 | 7 | 3.5 | 85 | 35 | 1.1 | 100000 | 2570 |
| 292 | Fe90.9Nb6B3C0.1 | amorphous | 18 | 7 | 3.9 | 81 | 36 | 5.9 | 24000 | 1300 |
| 293 | Fe93.06Nb2.97B2.97C1 | amorphous | 23 | 7 | 3.6 | 82 | 37 | 4.8 | 30000 | 1600 |
| 294 | Fe94.05Nb1.98B2.97C1 | amorphous | 12 | 7 | 3.4 | 90 | 37 | 4.9 | 56000 | 2100 |
| 295 | Fe90.9Nb1.98B2.97C4 | amorphous | 12 | 8 | 3.6 | 87 | 35 | 3.1 | 64000 | 2300 |
| 296 | Fe90.9Nb3B6C0.1 | amorphous | 16 | 7 | 3.7 | 82 | 34 | 5.8 | 28000 | 1400 |
| 297 | Fe94.5Nb3B2C0.5 | amorphous | 14 | 8 | 3.9 | 84 | 38 | 4.8 | 23000 | 1380 |
| 298 | Fe839Nb7B9C0.1 | amorphous | 22 | 6 | 3.0 | 81 | 39 | 3.6 | 42000 | 1860 |
| 299 | Fe80.8Nb6.7B8.65C3.85 | amorphous | 23 | 6 | 2.9 | 82 | 40 | 2.8 | 79000 | 2300 |
| 300 | Fe77.9Nb14B8C0.1 | amorphous | 24 | 6 | 3.0 | 80 | 32 | 7.6 | 23000 | 1700 |
| 301 | Fe75Nb13.5B7.5C4 | amorphous | 15 | 7 | 3.7 | 82 | 39 | 3.2 | 64000 | 2130 |
| 302 | Fe78Nb1B17C4 | amorphous | 12 | 7 | 3.4 | 89 | 41 | 11.2 | 34000 | 1400 |
| 303 | Fe78Nb1B20C1 | amorphous | 22 | 7 | 3.6 | 83 | 44 | 10.3 | 23000 | 1390 |

It turned out that magnetic properties or so of a sample can be anticipated by analyzing network structures of predetermined elements in the sample. It also turned out that the analysis of the network structure of specific elements in a sample can determine optimal manufacture conditions and develop a product having optimal characteristics. It also turned out that a correlation between a degree of network formation and characteristics (e.g., magnetic properties) of a sample can be analyzed in more detail by digitizing the degree of network formation and analyzing it.

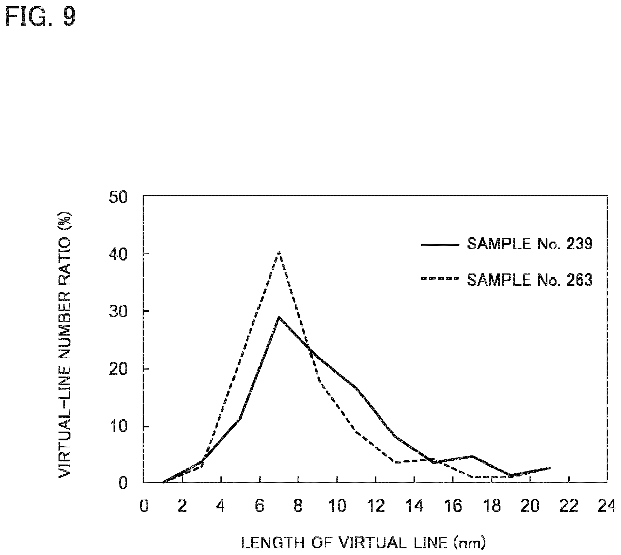

Incidentally, a ratio of the number of virtual lines to each length of virtual line between a maximum point and a maximum point was graphed with respect to Sample No. 239 of Table 6 and Sample No. 263 of Table 3. FIG. 9 is graphed results. In FIG. 9, a horizontal axis represents a length of virtual lines, and a vertical axis represents a ratio of the number of virtual lines. In the preparation of the graph of FIG. 9, it is considered that a virtual line having a length of 0 or more and less than 2 nm has a length of 1 nm, a virtual line having a length of 2 nm or more and less than 4 nm has a length of 3 nm, and a virtual line having a length of 4 nm or more and less than 6 nm has a length of 5 nm. The same shall apply hereafter. Then, a ratio of the number of virtual lines to a length of each virtual line is plotted, and the graph was prepared by connecting the plotted points with straight lines. Incidentally, the horizontal axis of FIG. 9 has a unit of nm.

FIG. 9 shows that the Fe-Si-M-B-Cu-C based composition shown in Table 6 has a larger variation of the length of virtual lines than that of the Fe-M-B-C based composition shown in Table 7.

### (Experiment 6)

Sample No. 304 to Sample No. 307 were manufactured in the same manner as Experiment 3. These samples were analyzed in the same manner as Experiment 4 with respect to a virtual-line total distance, a virtual-line average distance, and a virtual-line standard deviation. Moreover, an existence ratio of virtual lines having a length of 4 to 16 nm and a volume ratio of a Fe network composition phase were analyzed. FIG. 8 shows the results.

**Table 8**

| Sample No. | Composition | Gas temperature (°C) | Vapor pressure (hPa) | Network structures | | | | | Coercivity (A/m) |
|---|---|---|---|---|---|---|---|---|---|
| | | | | Virtual-line total distance (mm/µm³) | Virtual-line average distance (nm) | Virtual-line standard deviation (nm) | Existence ratio of 4 to 16 nm virtual lines (%) | Fe network composition phase (vol%) | |
| 304 | Fe73.5Cu1Nb3Si13.5B9 | 30 | 25 | <1 | - | - | - | - | **38** |
| 305 | Fe73.5Cu1Nb3Si13.5B9 | 100 | 4 | 11 | 9 | 4.2 | 81 | 35 | 24 |
| 306 | Fe84Nb7B9 | 30 | 25 | 6 | 5 | 2.8 | 56 | - | **280** |
| 307 | Fe84Nb7B9 | 100 | 4 | 14 | 9 | 4.2 | 82 | 36 | 98 |

As shown in Table 8, even if a sample was an alloy powder, a network structure was also digitized by digitizing a virtual-line total distance, a virtual-line average distance, a virtual-line standard deviation, and an existence ratio of virtual lines having a length of 4 to 16 nm.

It turned out that magnetic properties or so of a sample can be anticipated by analyzing network structures of predetermined elements in the sample. It also turned out that the analysis of the network structure of specific elements in a sample can determine optimal manufacture conditions and develop a product having optimal characteristics. It also turned out that a correlation between a degree of network formation and characteristics (e.g., magnetic properties) of a sample can be analyzed in more detail by digitizing the degree of network formation and analyzing it.

That is, a favorable Fe network was formed by appropriately carrying out a heat treatment against the complete amorphous powders in Sample No. 305 and Sample No. 307. Sample No. 304 and Sample No. 306, which had a too low gas temperature of 30°C and a too high vapor pressure of 25 hPa, however, had short virtual-line total distance and virtual-line average distance after the heat treatment, no favorable Fe composition network, and a high coercivity.

When comparing the samples shown in Table 8, it was found that an amorphous soft magnetic alloy powder was obtained by changing a gas spray temperature, a virtual-line total distance and a virtual-line average distance increased in the same manner as a ribbon by carrying out a heat treatment against the amorphous soft magnetic alloy powder, and a favorable Fe composition network structure was obtained. It was also found that coercivity tended to be small by having a network structure of Fe in the same manner as the ribbons of Experiments 4 and 5.

### Numerical References

- 10: unit grid
- 10a: maximum-point grid
- 10b: adjacent grid
- 20a: high-concentration region
- 20b: low-concentration region

## Claims

1. An analysis method of a composition network topology structure, comprising the steps of:
obtaining a three-dimensional body for analysis capable of being used for three-dimensional measurement of a concentration distribution of a specific element contained in a sample within a predetermined measurement range;
dividing the three-dimensional body for analysis into unit grids composed of a plurality of finer three-dimensional bodies;
obtaining an amount of the specific element contained in each of the unit grids;
obtaining maximum-point grids respectively having a largest amount of the specific element among adjacent unit grids; and
quantifying the composition network topology structure of the specific element owned by the sample in relation to the maximum-point grids contained in the three-dimensional body for analysis.

2. The analysis method of the composition network topology structure according to claim 1, further comprising the steps of:
forming virtual connection lines by linking a plurality of centers of the maximum-point grids existing inside the three-dimensional body for analysis;
forming final virtual connection lines by deleting the virtual connection lines being crossed based on a predetermined rule; and
determining the number of the final virtual connection lines linking each of the maximum-point grids as a coordination number,
wherein the composition network topology structure of the specific element owned by the sample is quantified based on the coordination number.

3. The analysis method of the composition network topology structure according to claim 1, further comprising the steps of:
forming virtual connection lines by linking a plurality of centers of the maximum-point grids existing inside the three-dimensional body for analysis;
forming final virtual connection lines by deleting the virtual connection lines being crossed based on a predetermined rule; and
obtaining data of the virtual connection lines including at least one of a total length of the final virtual connection lines linking the maximum-point grids inside the three-dimensional body for analysis, an average distance of the final virtual connection lines, a standard deviation of the final virtual connection lines, and an existence ratio of the final virtual connection lines within a predetermined length,
wherein the composition network topology structure of the specific element owned by the sample is quantified based on the data of the virtual connection lines.

4. The analysis method of the composition network topology structure according to claim 2 or 3, further comprising a step of obtaining an average value of the amount of the specific element contained in each of the unit grids with respect to the entire three-dimensional body for analysis,
wherein the entire three-dimensional body for analysis is divided into a high-concentration region having continuous unit grids whose amount is larger than a threshold value determined as the average value and a low-concentration region having continuous unit grids whose amount is equal to or smaller than the threshold value, and
the step of deleting the virtual connection lines deletes virtual connection lines passing through the low-concentration region.

5. An analysis program configured to implement the analysis method of the composition network topology structure according to any of claims 1 to 4 at the time of implementation in a programmable computer.
